# EUROPEAN PATENT SPECIFICATION

(11) **EP 3 263 177 B1**
(45) Date of publication and mention of the grant of the patent: **26.06.2019**
(21) Application number: 17169532.3
(22) Date of filing: 04.05.2017
(51) Int. Cl.: A61N 5/06

(54) **SKIN TREATMENT DEVICE**
HAUTBEHANDLUNGSVORRICHTUNG
DISPOSITIF DE TRAITEMENT DE LA PEAU

(30) Priority: 27.06.2016 EP 16176379
(43) Date of publication of application: 03.01.2018
(73) Proprietor: Braun GmbH, 61476 Kronberg im Taunus (DE)
(72) Inventor: Beerwerth, Frank, 61476 Kronberg (DE); Dadic, Dalibor, 61476 Kronberg (DE); Heinemann, Felix, 61476 Kronberg (DE); Bielfeldt, Uwe, 61476 Kronberg (DE)
(74) Representative: Schneider, Stefan Michael

(56) References cited:
- WO-A2-2007/016634
- US-A1- 2005 055 070
- US-A1- 2005 231 983
- US-A1- 2011 098 789
- US-A1- 2013 060 309

## Description

### FIELD OF THE INVENTION

The present invention is concerned with a skin treatment device, for example a hand-held hair removal device. The device may comprise a handle with an energy supply, an application head with at least one heat source comprising at least one semiconductor light source, and at least one heat sink comprising cooling fins.

### BACKGROUND OF THE INVENTION

It is known to use skin treatment devices using semiconductor light sources for hair removal. For example, EP 1 771 121 B1 discloses a radiation device for use in an epilator comprising LEDs as a light source.

Every light based device inherently generates heat and the light source heats up. High temperatures of the light source may lead to a performance loss. Thus, an efficient cooling system is necessary to get a high performance light based hair removal device. Heat conductors in cooling systems spread and conduct heat quickly away from a heat source to cooling fins. Known cooling systems use for example a solid copper or aluminum bar for conducting heat from a heat source to cooling fins. However, this increases the weight of a skin treatment device, which is undesired. In addition, efficient use of such cooling systems is limited to transferring heat over short distances only which also limits efficiency of the light output power.

Further, US 2005/0231983 A1 discloses an LED curing device having a rigid body with an end tip comprising the LED and a fan located at an opposite end of the body. A heat pipe extending through the body is bonded or glued with one end to the LED and with an opposite end to a heat sink near the fan. The fan blows air over the heat sink.

It is an object of the present disclosure to provide skin treatment device with an improved heat transfer system.

### SUMMARY OF THE INVENTION

The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes. A skin treatment device is provided comprising a handle with an energy supply, an application head with at least one heat source comprising at least one semiconductor light source, and at least one heat sink comprising cooling fins, wherein the heat sink is located remote from the heat source within the handle, and wherein the heat source is thermally connected to the heat sink by means of a heat pipe heat transfer system. In more detail, the heat transfer system may comprise a hermetically sealed tubular body containing a working medium, wherein the pressure within the tubular body is chosen such that the working medium is a saturated liquid at room temperature and may be vaporized by the heat source. A wick structure may be located in the tubular body exerting a capillary action on the working fluid, i.e. for transporting the liquid from the condensation area (heat sink) to the evaporation area (heat source). Wick structures suitable for heat pipes include sintered metal powder, screen, and grooved wicks, which have a series of grooves parallel to the pipe axis. Depending on the geometry of the tubular body, provision of a wick is not required. For example, a heat pipe may have a configuration and/or coating provided on the inner walls for providing a capillary action. High heat conductance in conjunction with the fact that heat pipes are lightweight enables the possibility to spatially separate heat source and heat sink over higher distances.

In accordance with one aspect the application head may be sealed, e.g. hermetically, without openings to the ambience and may comprise at least one air channel adapted for guiding air to the at least one semiconductor light source. The at least one air channel may have at least one inlet opening which is located in the handle and at least one outlet opening which is located in the handle.

In addition or as an alternative, in accordance with a further aspect the application head may be pivotably connected to the handle.

### BRIEF DESCRIPTION OF THE DRAWINGS

- Figure 1: shows a sectional view of a skin treatment device according to a first embodiment;
- Figure 2: shows a sectional view of a skin treatment device according to a second embodiment;
- Figure 3: shows a partial sectional view of a skin treatment device according to a third embodiment;
- Figure 4: shows a partial sectional view of a skin treatment device according to a fourth embodiment;
- Figure 5: shows a perspective view of a heat transfer system according to the invention;
- Figures 6a-c: show different positions of the application head relative to the handle;
- Figure 7: shows an alternative to Figures 6a-c;
- Figure 8a: shows a partial top view of a detail of a skin treatment device according to a fifth embodiment;
- Figure 8b: shows a perspective view of the heat transfer system of Figure 8a;
- Figures 9a, b: show in partial perspective and sectional views details of a skin treatment device according to a sixth embodiment;
- Figures 10a, b: show in partial perspective and sectional views details of a skin treatment device according to a seventh embodiment;
- Figures 11a, b: show in partial perspective and sectional views details of a skin treatment device according to an eighth embodiment;
- Figure 12: show details of a hinge in a skin treatment device according to a further embodiment;
- Figures 13a, b: show details of a hinge in a skin treatment device according to a further embodiment; and
- Figures 14a, b: show details of a hinge in a skin treatment device according to a further embodiment.

### DETAILED DESCRIPTION OF THE INVENTION

Heat pipes in different base shapes of the tubular body, for example round or flat, are suitable for use in the skin treatment device. In particular, the heat pipes may have a rectangular shape. The tubular body of the heat transfer system may be made from a thermally conductive material, especially copper, steel or aluminum. The working medium of the heat transfer system may be a fluid, like a refrigerant, for example water, ammonia or 1,1,1,2-Tetrafluoroethane (R-134a).

The energy supply may comprise an energy storage, like a battery, in particular a rechargeable battery, located in the handle. In addition or alternatively, the energy may be provided via a mains cable. Optionally, the heat source is electrically connected to the energy supply by means of the tubular body of the heat pipe heat transfer system.

The heat source of the skin treatment device may comprise light source suitable for hair removal. For example, the heat source may comprise a vertical-cavity surface-emitting laser (VCSEL) array, a vertical external cavity surface-emitting laser (VECSEL) array, a light emitting diode (LED) array and/or an organic light emitting diode (OLED) array.

The heat sink of the skin treatment device may comprise an appliance for increasing heat transfer from the heat source. For example at least one cooling fan may be provided to raise heat dissipation. In addition or alternatively, at least one thermoelectric element may be provided.

The application head may be sealed, in particular the head may be mechanically sealed without any openings. With such an environmental encapsulated head design the head is protected against environmental influences like dust, humidity, water, or lotion. In this case, the heat sink, including an optional fan, the cooling air inlets and outlets may be completely placed in the handle.

For some applications it may be desirable if the application head is rigidly connected to the handle. In addition, the use of, for example, rigid copper material for the tubular body of the heat pipe sets limits movements of the head relative to the handle. However, it is possible to bend heat pipes in required shapes. This characteristic enables more industrial design freedom between head and handle. The angle between the center line of the head and the center line of the handle may be chosen ± 60°. This allows an exposed head with a fixed angle between handle and head. Handling of the hand-held device improves, due to design freedom to get an optimal angle between handle and head for best intended device usability.

Alternatively, the application head may be pivotably connected to the handle. For example, heat pipes with an additional flexible segment enable designing a device with exposed swivel head. Heat conductors with an additional flexible segment enable designing a device with exposed swivel head. The head may be mechanically connected to the device body (handle) via a constant torque hinge, for example as typically used for notebook displays. This hinge separates the heat conductive means from the mechanical means. The constant torque hinge allows changing the angle while maintaining sufficient rigidity of the device for use without unintended change of the head-body angle. When providing a swiveling connection the heat conductivity of the connection is very important. A typical mechanical hinge even if made of copper or alumina may not have sufficient heat conductivity to meet the requirements for a device with extensive heat load within the device head.

An important aspect is the solution how to enable sufficient heat transport from the heat source in the application head to the heat sink in the handle or body while having a swiveling connection between both parts. A heat pipe may be made flexible by inserting a flexible segment between the heat source and heat cooling fins section. The flexible segment accommodates any relative motion between heat source and heat sink. An example for such a flexible segment is a flexible bellows section of a heat pipe arranged between the application head and the handle. As a further example, a flexible segment may comprise a highly heat conducting graphite foil, e.g. a pyrolytic graphite sheet (PGS), between the application head and the handle, e.g. connected to the heat source part and the heat sink part of the cooling system. Flexible heat pipes work in principle equivalent to the rigid heat pipes. This additional feature increases the usability of the device, because head orientation to skin adapts better to body shape while usage.

In addition or as an alternative, the application head may be pivotably connected to the handle by means of a heat conducting hinge having a spindle and a jack. The spindle may comprise a portion of the tubular body of a heat transfer system and the jack may comprise a portion of the tubular body of a heat transfer system or a pyrolytic graphite sheet (PGS). In other words, the heat transfer system may comprise a first heat pipe being part of the spindle and a second heat pipe being part of the jack. As an alternative, a heat pipe is part of the spindle and a pyrolytic graphite sheet is part of the jack.

According to one aspect, the application head of the skin treatment device further comprises a transparent or translucent skin contact window. Such a skin contact window prevents direct contact of the user's skin to the light source and further prevents environmental influences like dust, humidity, water, or lotion.

During operation a light source module, for example a LED light source module, may generate up to 75W heat power and the surface of the light source module could heat up to about 80°C. A part of the heat power is heating up the skin contact window. The higher the skin contact window temperature is the more uncomfortable is the usage for the user. To prevent high temperatures at outside surface of the skin contact window, active cooling of the skin contact window and/or thermally decoupling of the window from the heat source is desirable.

In this respect, a first side of the at least one semiconductor light source may be thermally connected to the heat transfer system and a second side of the at least one semiconductor light source which is opposite the first side may be located within the application head at a position and orientation permitting light to be emitted from the at least one semiconductor light source through the skin contact window. In other words, the heat pipe heat transfer system is arranged such that heat is transferred from the side facing away of the skin contact window from the heat source, i.e. the semiconductor light source.

In addition or as an alternative, the skin contact window may be thermally connected to the heat transfer system or it may be thermally connected to an additional heat transfer system. The additional heat transfer system may be a heat pipe heat transfer system which comprises for example a hermetically sealed tubular body containing a working medium wherein the pressure within the tubular body is chosen such that the working medium is a saturated liquid at room temperature which may be vaporized by the heat source. In other words, the skin contact window cooling design may comprise connection of the glass or ceramic window to a separate second heat pipe with a heat conductive frame, for example pre-stamped and/or with an applicable heat conductive gap filler, e.g. filled silicon rubber or graphite based filler). The window may be made of a good heat conducting material, like ceramic, sapphire or the like. Within the handle of the skin treatment device, the semiconductor light source and the skin contact window may each have a separate heat sink, for example separate cooling fins. As an alternative, the semiconductor light source and the skin contact window may share one common heat sink. Two different fans may be provided for separate heat sinks. However, even with two separate heat sinks, it is preferred to provide only one single fan which serves both cooling systems.

The skin contact window may be directly connected with a heat transfer system. However, for providing a homogeneous and higher performance heat flow the skin contact window may be thermally connected to the heat transfer system or to the additional heat transfer system by means of a thermoelectric cooler. For example, this skin contact window cooling system comprises a Peltier element placed between the heat pipe and the inner side of the skin contact window. A Peltier element is an active heat conduction element which enables homogeneous and high performance heat flow from low to high temperature sections. Provision of a thermoelectric cooler, like a Peltier element, facilitates heat transfer from the light source and from the skin contact window using only one single heat pipe to cool both, the LED module or the like light source and the skin contact window at lower temperature at the same time.

In addition to transferring heat from the skin contact window to the handle or as an alternative, the application head may comprise cooling fins which are thermally connected to the skin contact window. For example, the skin contact window cooling system comprises a head housing made of a heat conductive material, e.g. aluminum or specific filled plastic, which is connected to the skin contact window directly or via a conductive frame. In other words, the application head housing may serve as a heat sink, for example as a heat capacitor and/or with additional cooling fins. Heat sink fins can be integrated in the application head housing, i.e. the application head housing and cooling fins are one component part. Alternatively, cooling fins may be fitted at the rear side of the head that is the side facing away from the skin contact window. This design supports an encapsulated application head design.

Further or as an alternative, the application head may comprise at least one air channel adapted for guiding air through a gap between the at least one semiconductor light source and the skin contact window. The skin contact window cooling system may comprise an air channel between the application head and the handle. A function of the channel is to suck heated air from the gap area between the light source and the skin contact window by means of a fan which may be placed in the handle. The working principle of this fan is to suck air from one side (inlet side) and to create overpressure at the opposite side (outlet side). At the suction or inlet side the fan generates a negative pressure, i.e. a vacuum. With the fan located in the handle and the handle connected to the application head via an air channel, vacuum generated by the fan in the handle sucks in air of ambient pressure from the application head area via the air channel, thereby generating an air exchange from the application head to the handle. This working principle requires a connection of the application head to the ambient pressure to supply the head with fresh cooling air.

In general, the at least one air channel may comprise at least one inlet opening which is located in the applicator head and/or in the handle and at least one outlet opening which is located in the applicator head and/or in the handle. For example, a fresh air inlet for window cooling and/or interruption of heat flow to the window from the light source is located in the handle which is directly connected to the application head. In some embodiments, the application head may include air guiding walls to lead the air between the light source and the skin contact window. A second channel connection to the handle may suck the heated air due to vacuum generated by the fan in the handle. Due to sealed head design, it is possible to use a lotion or the like liquid with the skin treatment device. Alternatively, a fresh air inlet for head cooling is located in the application head itself. For example, the application head includes air support walls to lead the air between the light source and the skin contact window. In some embodiments, only one cooling channel between the application head and the handle may be sufficient. Due to this non-encapsulated head design, the use of a lotion or the like liquid together with the device is limited. The cooling air channels may be integrated in the handle and/or in the head housing or may be provided as an extra part, e.g. extra build-in pipe channels.

Still further, the applicator head may comprise a fan adapted to pass air through the air channel. In more detail, the skin contact window cooling system may comprise a non-encapsulated design of the application head with small openings and a small fan integrated in the head. Continuous exchange of fresh air between the skin contact window and the light source module prevents that the heat emitted by the light source module heats up the skin contact window to a temperature level which is unpleasant for the user. Usability of lotion or the like is limited due to the non-encapsulated head.

The light output area may be designed such that a window is provided which does not directly contact a user's skin. For example, an additional cap, which is placed in front of the light output area, i.e. a window, prevents during usage a contact between the skin and the window. According to one aspect, the cap has no contact to the window, except some possible fixation contact points. This provides a small air gap between the skin contact cap and the window which prevents heat transfer from the window to the skin spacer cap.

Such a skin spacer cap may reduce heat transfer from a heated window to the skin due to a reduced contact surface size to the skin. In addition, the cap may keep the skin on distance from the heated window. It may further serve as a light guide for the emitted light. Provision of such a cap may result in that no additional cooling of the window may be required.

An additional gel-cushion, a gel-cushion attachment, may be placed in front of the light output window. The gel may have a high light-transmissivity for visible and NIR-light and is encased by a flexible and transparent foil. The attachment may have no contact to the window, except of some fixation contacts. A small gap between the gel-cushion attachment and the window prevents or significantly reduces the heat transfer of the warm window to the gel-cushion. A benefit of the gel-cushion is to improve the adaptation of the head to body shapes due flexible properties of the gel-cushion while device usage and the possibility to use lotion or the like liquids together with the skin treatment device.

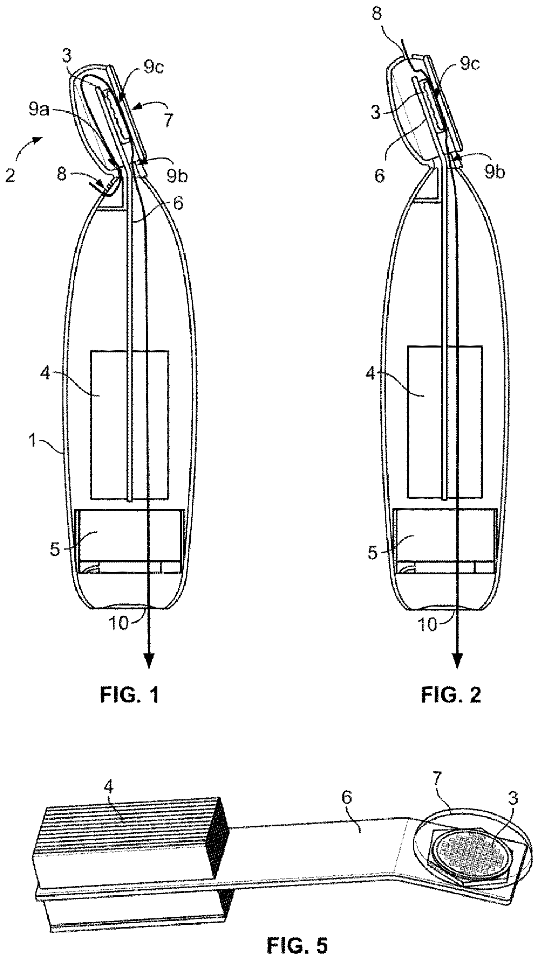

The skin treatment device as shown in the Figure 1 comprises handle 1 and an application head 2. The application head 2 comprises a light source in the form often an LED module 3. The light based device is suitable for skin treatment, especially for applications that need a high intensity light source, and if the light delivery system is desired to be small and light. Possible applications include light based hair removal, light based skin rejuvenation via bio-photo modulation and acne treatment.

The application head 2 may be designed to be a separate component part which is split from the handle 1. This results in a better application head adjustment to different body shapes. Further, an environmental encapsulated head design is feasible. The compact size of the application head improves the usage precision for small treatment areas (e.g. face and bikini) by enhancing the visibility of the skin to be treated.

In the skin treatment device of Figure 1, an energy supply, for example an energy source in the form of a rechargeable battery (not shown), is provided in the handle 1. Further, a heat sink in the form of cooling fins 4 and a fan 5 are provided in the handle 1 allowing transferring heat generated by the light source to the heat sink. Due to high temperatures of the light source resulting in a performance loss of the skin treatment device, efficient heat transfer is desirable.

The LED module 3 is thermally connected to the heat sink 4 by means of a heat pipe 6. The heat pipe 6 comprises a tubular body of a flat and substantially rectangular shape which is hermetically sealed. A working medium is located within the tubular body with the pressure of the working medium within the tubular body being chosen such that the working medium is a saturated liquid at room temperature and may be vaporized by the heat source that is of the light source 3. As can be seen for example from Figure 5 the heat pipe 6 has the light source 3 and the heat sink 4 directly applied to the outer surface of the tubular body. Optionally, the tubular body of the heat pipe 6 further electrically connects the energy source with the light source.

In the embodiment of Figure 1 the application head 2 is provided with a skin contact window 7 which contacts the user's skin during use of the skin treatment device. The application head 2 is an encapsulated together with the skin contact window 7 to prevent the head 2 against environmental influences like dust, humidity, water, or lotion.

An air channel is provided in the skin treatment device, which air channel comprises an inlet 8 located in the handle 1, a passage 9a from the handle 1 to the head 2, a further passage 9b from the head 2 to the handle 1, and an outlet 10. Thus, ambient air may be sucked in by fan 5 via the inlet 8, floating about the light source 3 and through a channel 9c between the light source and the skin contact window 7, back into the handle 1 and through fan 5 to outlet 10. In other words, fan 5 not only supplies cooling air to fins 4 but also for cooling skin contact window 7.

An alternative design is depicted in Figure 2. In this embodiment, the inlet 8 is provided directly in the application head 2. Thus, cooling air is sucked in by fan 5 through inlet 8, passes through the channel 9c between the light source and the skin contact window 7, passes along cooling fins 7 and is finally exhausted through outlet 10.

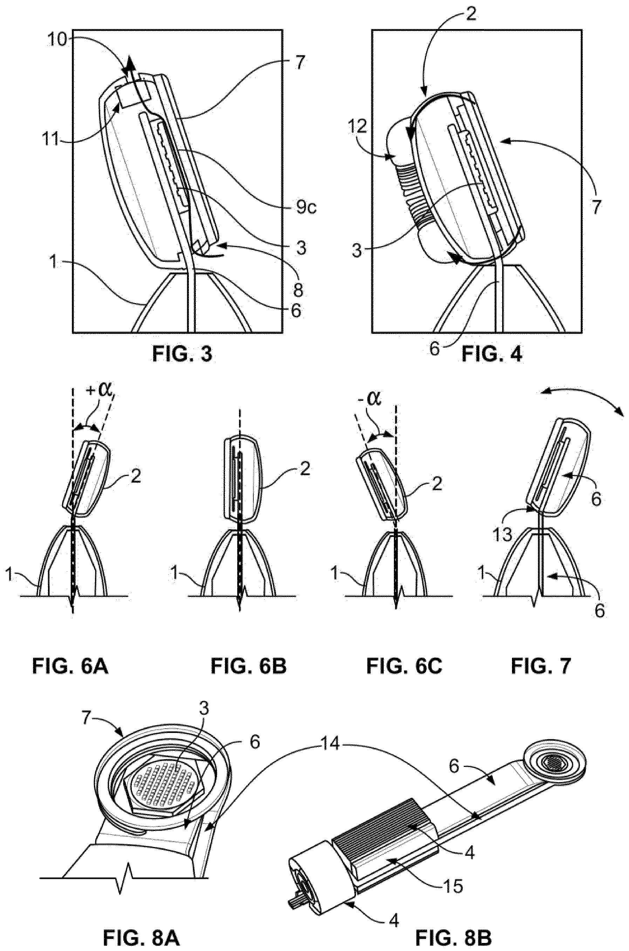

A further alternative is depicted in Figure 3. In this embodiment the application head 2 is provided with a separate fan 11. An inlet 8 and an outlet 10 are located in the application head 2 such that cooling air may pass through the application head 2 and through the channel 9c between the light source and the skin contact window. In this embodiment an air passage between the handle 1 and the application head 2 is not required. An air inlet and an air outlet may be provided in the handle 1 together with fan 5 to supply cooling air to fins 4 as depicted in Figures 1 and 2.

Still further embodiment is depicted in Figure 4. In this embodiment the application head 2 is provided as a heat sink for the skin contact window 7. In this respect cooling fins 12 may be provided at the side facing away from the skin contact window 7. Heat may be transferred from the skin contact window 7 via the housing of the application head 2 to the cooling fins 12 as indicated by arrows in Figure 4.

Due to the relatively rigid structure of heat pipe 6, the application head 2 may be fixed with respect to the handle 1. Nevertheless, it is possible to position the application head 2 with respect to the handle 1 in different ways to get an optimal angle between of the application head 2 and the handle 1 for the best intended usability of the handheld skin treatment device. Figures 6a to 6c show exemplary embodiments of different angles between the application head 2 and handle 1. For example, the angle α between the center axis of the handle 1 and the application head 2 may be positive as shown in Figure 6a, or 0 as shown in Figure 6b, or negative as shown in Figure 6c.

As an alternative, heat pipe 6 may be provided with an additional flexible segment 13 as depicted in Figure 7. This flexible segment 13 permits swiveling between the application head 2 and the handle 1. The flexible segment 13 may comprise a flexible tube, a corrugated pipe and/or a heat conducting graphite foil.

In more detail, the flexible segment 13 may be a swiveling head design with a flexible heat pipe 6. Heat pipes may be made flexible by inserting a flexible bellows section between the upper and lower part of the heat conductor/heat pipe. Such a flexible heat pipe is available for example from the company Aavid Thermalloy, LLC under the name Thermacore flexible heat pipe. This additional feature of a swiveling application head 2 increases the usability of the device, because adjustment of the head-body angle allows improved handling on difficult to reach body areas.

As an alternative, the flexible segment 13 for the swiveling head design may comprise a PGS (Pyrolytic Graphite Sheet). Such a PGS has a very high heat conductivity within the plane of the material. The heat conductivity can be up to 5 times higher than the heat conductivity of pure copper. In addition, the PGS material allows bending with very narrow bending radius and high number of bending cycles. Such a PGS is available from the company Panasonic under the name PGS graphite sheet.

To improve heat transfer from the skin treatment window 7, the skin treatment window 7 may be thermally connected with a separate heat transfer system in the form of a heat pipe 14 as shown in Figures 8a and 8b. The additional heat pipe 14 may be connected with separate cooling fins 15, for example located adjacent to cooling fins 4 of heat pipe 6. Fan 5 which supplies cooling air to fins 4 may also supply cooling air to fins 15.

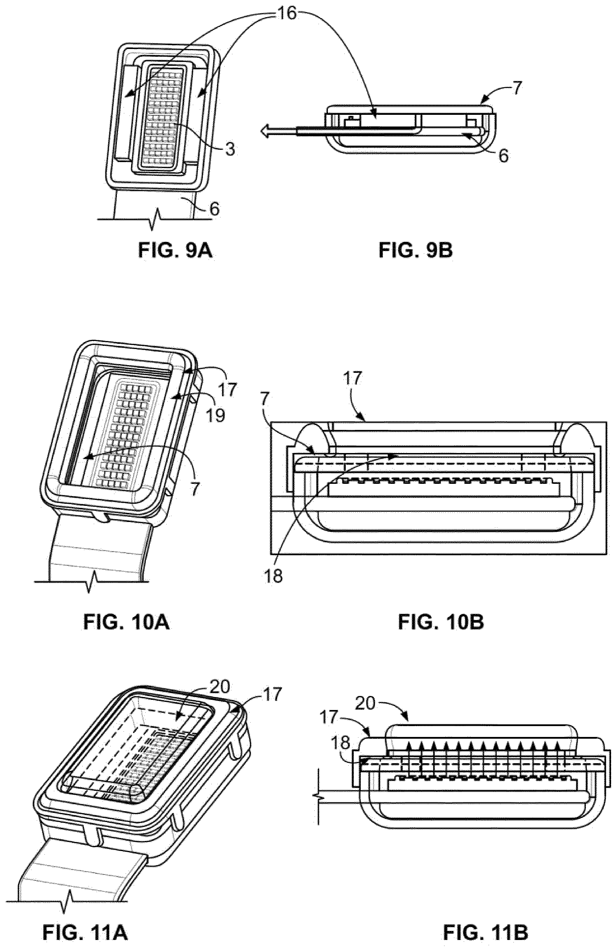

As an alternative, the skin contact window 7 may be provided with a cooling system transferring heat from the skin contact window 7 to the cooling fins 4 by means of heat pipe 6. In this embodiment it is preferred to provide a thermoelectric cooler, for example a Peltier element 16 as shown in Figures 9a and 9b, interposed between the skin cooling window 7 and the heat pipe 6.

For some applications it may be desirable to prevent direct contact between a user's skin and a window 7 of the device. As an alternative to a skin contact window 7 a light output window cap in the form of a skin spacer cap 17 is provided in the embodiment depicted in Figures 10a, 10b. The skin spacer cap 17 is positioned at the application head 2 at the side of a window 7 facing away from the light source. The contact area between the skin spacer cap 17 and the window 7 and further component parts of of the application head 2 may be minimized by having only small fixation contacts. In addition, a small gap 18 may be provided between the skin spacer cap 17 and the window 7. The heat transfer from the light source 3 to the skin of a user may be further minimized by reducing the contact area 19 of the skin spacer cap 17 and the user's skin.

In addition or as an alternative, a gel cushion 20 may be provided between the window 7 and the user's skin as depicted in Figures 11a, 11b. The additional gel cushion 20 is placed in front of the light output window 7. The gel has a high light-transmissivity for visible and NIR-light and is encased by flexible and transparent foil. The attachment, for example the skin spacer cap 17, has no contact to the window 7, except for some fixation contacts. A small gap 18 between gel cushion attachment and window 7 reduces the conductance of the warm window 7 to the gel cushion 20.

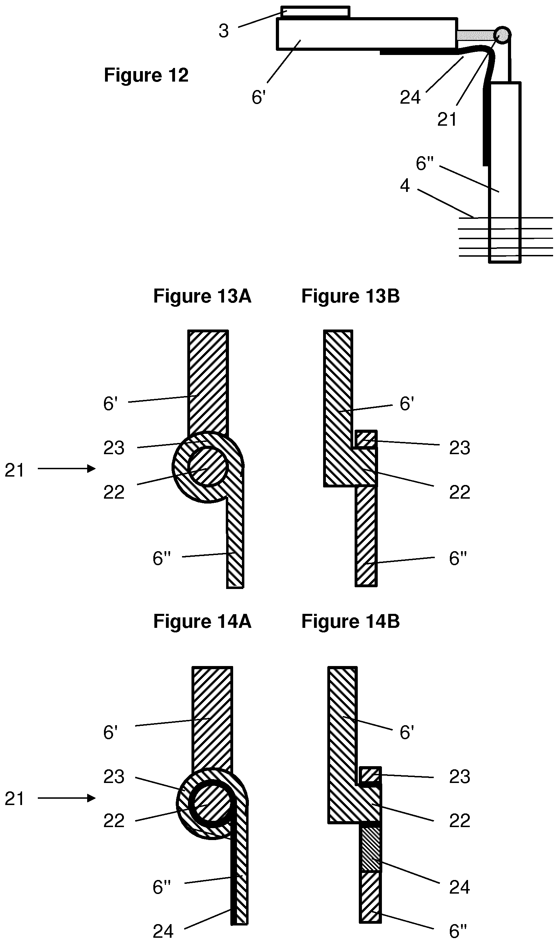

Figure 12 depicts a schematic embodiment of a detail of a skin treatment device with a light source 3 which is a heat source and cooling fins 4 which are the heat sink. The light source 3 and the cooling fins 4 are connected to each other by means of a heat transfer system 6 comprising a first heat conductor 6' and a second heat conductor 6" which are connected via a hinge 21, for example a torque hinge. The hinge may comprise a spindle 22 and the jack 23 as depicted in Figures 13a to 14b with the spindle 22 being connected to the second heat conductor 6" and the jack 23 being connected to the first heat conductor 6'. The first heat conductor 6' and the second heat conductor 6" are further thermally connected by means of a PGS 24 forming a flexible part 13 of the heat transfer system 6.

In the present embodiment, the first (upper) heat conductor 6' may be just a plate of a good heat conducting material, e.g. copper or alumina, a heat pipe 6 or directly an extended area of the PGS sheet 24. The heat from the heat source 3 in the head 2 is transferred via the upper heat conductor 6' to the flexible part 13 of the heat conductor which may be a PGS foil. The hinge 21 of Figure 12 mainly serves for mechanical stability, whereas heat conductivity of hinge 21 may be relatively insignificant.

An aspect of the embodiment of Figure 12 is that the length of the PGS foil 24 between the upper and the lower heat conductors 6', 6" is preferably low. It is desired to keep this length as low as possible, for example at a length below 10 mm, in order to keep the temperature difference over the length of the unsupported part of the foil 24 close to the hinge 21 low. The lower heat conductor 6" may also be directly the heat sink (copper, alumina with fins 4) or a heat pipe 6 to allow heat transfer to the remotely located heat sink 4.

Another option to transfer the heat from the head 2 to the body or handle 1 is using a specially designed hinge 21 which has the capability to transfer sufficient heat.

Figures 13a and 13b depict an alternative embodiment of a hinge 21 suitable for swiveling application head 2 with respect to the handle 1 of the skin treatment device while allowing sufficient heat transfer. Figures 13a and 13b show the hinge 21 build of two heat pipes each forming one of the upper and the lower heat conductors 6', 6". The cylindrical contact area between spindle 22 and jack 23 transfers the heat from the spindle 22 to the jack 23. To increase the heat transfer heat conducting lubricant may be used.

Figures 14a and 14b depict a further alternative embodiment of a hinge 21 suitable for swiveling application head 2 with respect to the handle 1 of the skin treatment device while allowing sufficient heat transfer. Figures 14a and 14b show a design using on the jack side 23 a PGS 24 as a heat conductor. The spindle 22 is again made using heat pipe technology.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

### Reference Numerals

- 1: handle
- 2: head
- 3: light source
- 4: fins
- 5: fan
- 6: heat pipe
- 6', 6": heat conductor
- 7: window
- 8: inlet
- 9a-c: channel
- 10: outlet
- 11: fan
- 12: fins
- 13: flexible segment
- 14: heat pipe
- 15: fins
- 16: thermoelectric cooler
- 17: skin spacer cap
- 18: gap
- 19: contact area
- 20: gel cushion
- 21: hinge
- 22: spindle
- 23: jack
- 24: PGS

- α: angle

## Claims

1. A skin treatment device comprising
a handle (1) comprising an energy supply,
an application head (2) comprising at least one heat source comprising at least one semiconductor light source (3), and
at least one heat sink comprising cooling fins (4),
wherein the heat sink is located remote from the heat source within the handle (1), wherein the heat source is thermally connected to the heat sink by means of a heat transfer system (6, 6', 6") which comprises at least one hermetically sealed tubular body containing a working medium, wherein the pressure within the tubular body is chosen such that the working medium is a saturated liquid at room temperature and may be vaporized by the heat source,
and wherein the application head (2) is sealed without openings to the ambience **characterized in that** the application head (2) comprises at least one air channel (9a, 9b, 9c) adapted for guiding air to the at least one semiconductor light source (3), the at least one air channel (9a, 9b, 9c) having at least one inlet opening (8) which is located in the handle (1) and at least one outlet opening (10) which is located in the handle (1).

2. The skin treatment device according to claim 1, **characterized in that** the at least one air channel (9a, 9b, 9c) is adapted for guiding air through a gap (9c) between the at least one semiconductor light source (3) and a transparent or translucent skin contact window (7) of the application head (2).

3. The skin treatment device according to any of the preceding claims, comprising a skin contact window (7), **characterized in that** the skin contact window (7) is thermally connected to the heat transfer system (6) or is thermally connected to an additional heat transfer system (14) which comprises an hermetically sealed tubular body containing a working medium wherein the pressure within the tubular body is chosen such that the working medium is a saturated liquid at room temperature which may be vaporized by the heat source.

4. The skin treatment device according to claim 3, **characterized in that** the skin treatment device comprises a thermoelectric cooler (16) and the skin contact window (7) is thermally connected to the heat transfer system (6, 6', 6") or to the additional heat transfer system (14) by means of the thermoelectric cooler (16).

5. The skin treatment device according to any of claims 2 to 4, **characterized in that** the application head (2) comprises cooling fins (12) which are thermally connected to the skin contact window (7).

6. The skin treatment device according to any of the preceding claims, **characterized in that** the heat source is electrically connected to the energy supply by means of at least one tubular body of a heat transfer system (6, 6', 6"; 14).

7. The skin treatment device according to any of the preceding claims, **characterized in that** the heat sink further comprises at least one cooling fan (5).

8. The skin treatment device according to any of the preceding claims, **characterized in that** the applicator head (2) comprises a fan (11) adapted to pass air through the air channel (9a, 9b, 9c).

9. The skin treatment device according to any of claims 2 to 8, **characterized in that** a first side of the at least one semiconductor light source (3) is thermally connected to the heat transfer system (6, 6', 6") and that a second side of the at least one semiconductor light source (3) which is opposite the first side is located within the application head (2) at a position and orientation permitting light to be emitted from the at least one semiconductor light source (3) through the skin contact window (7).

10. The skin treatment device according to any of claims 2 to 9, **characterized in that** the application head (2) is pivotably connected to the handle (1) by means of a constant torque hinge (21).

11. The skin treatment device according to any of claims 2 to 10, **characterized in that** the application head (2) is pivotably connected to the handle (1) by means of a heat conducting hinge (21) having a spindle (22) and a jack (23), wherein the spindle (22) comprises a portion of the tubular body of a heat transfer system (6, 6', 6"; 14) and the jack (23) comprises a portion of the tubular body of a heat transfer system (6, 6', 6"; 14) and/or a pyrolytic graphite sheet (24).

12. The skin treatment device according to any of claims 2 to 11, **characterized in that** the at least one heat transfer system (6, 6', 6"; 14) comprises a pyrolytic graphite sheet (24) between the application head (2) and the handle (1).

13. The skin treatment device according to any of claims 2 to 11, **characterized in that** the at least one heat transfer system (6, 6', 6"; 14) comprises a flexible bellows section (13) between the application head (2) and the handle (1).

14. The skin treatment device according to any of the preceding claims, **characterized in that** the heat source comprises a vertical-cavity surface-emitting laser (VCSEL) array, a vertical external cavity surface-emitting laser (VECSEL) array, a light emitting diode (LED) array or an organic light emitting diode (OLED) array.

## Patentansprüche

1. Hautbehandlungsvorrichtung, die Folgendes umfasst:
einen Griff (1), der eine Energieversorgung umfasst,
einen Applikationskopf (2), der wenigstens eine Wärmequelle umfasst, die wenigstens eine Halbleiterlichtquelle (3) umfasst, und
wenigstens einen Wärmeabieiter, der Kühlrippen (4) umfasst,
wobei der Wärmeableiter entfernt von der Wärmequelle innerhalb des Griffs (1) angeordnet ist,
wobei die Wärmequelle thermisch mit dem Wärmeableiter mittels eines Wärmeübertragungssystems (6, 6', 6") verbunden ist, das wenigstens einen hermetisch abgedichteten röhrenförmigen Körper umfasst, der ein Arbeitsmedium enthält, wobei der Druck innerhalb des röhrenförmigen Körpers so gewählt ist, dass das Arbeitsmedium bei Raumtemperatur eine gesättigte Flüssigkeit ist und von der Wärmequelle verdampft werden kann,
und wobei der Applikationskopf (2) ohne Öffnungen gegenüber der Umgebung abgedichtet ist, **dadurch gekennzeichnet, dass** der Applikationskopf (2) wenigstens einen Luftkanal (9a, 9b, 9c) umfasst, der dafür angepasst ist, Luft zu der wenigstens einen Halbleiterlichtquelle (3) zu führen, wobei der wenigstens eine Luftkanal (9a, 9b, 9c) wenigstens eine Einlassöffnung (1) aufweist, die im Griff (8) angeordnet ist, und wenigstens eine Auslassöffnung (10) aufweist, die im Griff (1) angeordnet ist.

2. Hautbehandlungsvorrichtung nach Anspruch 1, **dadurch gekennzeichnet, dass** der wenigstens eine Luftkanal (9a, 9b, 9c) dafür angepasst ist, Luft durch einen Spalt (9c) zwischen der wenigstens einen Halbleiterlichtquelle (3) und einem durchsichtigen oder lichtdurchlässigen Hautkontaktfenster (7) des Applikationskopfes (2) zu führen.

3. Hautbehandlungsvorrichtung nach einem der vorstehenden Ansprüche, die ein Hautkontaktfenster (7) umfasst, **dadurch gekennzeichnet, dass** das Hautkontaktfenster (7) thermisch mit dem Wärmeübertragungssystem (6) verbunden ist oder thermisch mit einem zusätzlichen Wärmeübertragungssystem (14) verbunden ist, das einen hermetisch abgedichteten röhrenförmigen Körper umfasst, der ein Arbeitsmedium enthält, wobei der Druck innerhalb des röhrenförmigen Körpers so gewählt ist, dass das Arbeitsmedium bei Raumtemperatur eine gesättigte Flüssigkeit ist und von der Wärmequelle verdampft werden kann.

4. Hautbehandlungsvorrichtung nach Anspruch 3, **dadurch gekennzeichnet, dass** die Hautbehandlungsvorrichtung einen thermoelektrischen Kühler (16) umfasst, und das Hautkontaktfenster (7) thermisch mit dem Wärmeübertragungssystem (6, 6', 6") oder dem zusätzlichen Wärmeübertragungssystem (14) mittels eines thermoelektrischen Kühlers (16) verbunden ist.

5. Hautbehandlungsvorrichtung nach einem der Ansprüche 2 bis 4, **dadurch gekennzeichnet, dass** der Applikationskopf (2) Kühlrippen (12) umfasst, die thermisch mit dem Hautkontaktfenster (7) verbunden sind.

6. Hautbehandlungsvorrichtung nach einem der vorstehenden Ansprüche, **dadurch gekennzeichnet, dass** die Wärmequelle elektrisch mit der Energieversorgung mittels wenigstens eines röhrenförmigen Körpers eines Wärmeübertragungssystems (6, 6', 6"; 14) verbunden ist.

7. Hautbehandlungsvorrichtung nach einem der vorstehenden Ansprüche, **dadurch gekennzeichnet, dass** der Wärmeableiter ferner wenigstens einen Kühllüfter (5) umfasst.

8. Hautbehandlungsvorrichtung nach einem der vorstehenden Ansprüche, **dadurch gekennzeichnet, dass** der Applikationskopf (2) einen Lüfter (11) umfasst, der dafür angepasst ist, Luft durch den Luftkanal (9a, 9b, 9c) strömen zu lassen.

9. Hautbehandlungsvorrichtung nach einem der Ansprüche 2 bis 8, **dadurch gekennzeichnet, dass** eine erste Seite der wenigstens einen Halbleiterlichtquelle (3) thermisch mit dem Wärmeübertragungssystem (6, 6', 6") verbunden ist und sich eine zweite Seite der wenigstens einen Halbleiterlichtquelle (3), die sich gegenüber der ersten Seite befindet, innerhalb des Applikationskopfes (2) bei einer Position und Ausrichtung befindet, die ermöglichen, dass Licht aus der wenigstens einen Halbleiterlichtquelle (3) durch das Hautkontaktfenster (7) emittiert wird.

10. Hautbehandlungsvorrichtung nach einem der Ansprüche 2 bis 9, **dadurch gekennzeichnet, dass** der Applikationskopf (2) mittels eines Konstantmomentscharniers (21) schwenkbar mit dem Griff (1) verbunden ist.

11. Hautbehandlungsvorrichtung nach einem der Ansprüche 2 bis 10, **dadurch gekennzeichnet, dass** der Applikationskopf (2) mittels eines Wärmeleitscharniers (21), das eine Spindel (22) und eine Buchse (23) aufweist, schwenkbar mit dem Griff (1) verbunden ist, wobei die Spindel (22) einen Abschnitt des röhrenförmigen Körpers eines Wärmeübertragungssystems (6, 6', 6"; 14) umfasst, und die Buchse (23) einen Abschnitt des röhrenförmigen Körpers eines Wärmeübertragungssystems (6, 6', 6"; 14) umfasst und/oder eine pyrolytische Graphitlage (24) umfasst.

12. Hautbehandlungsvorrichtung nach einem der Ansprüche 2 bis 11, **dadurch gekennzeichnet, dass** das wenigstens eine Wärmeübertragungssystem (6, 6', 6"; 14) eine pyrolytische Graphitlage (24) zwischen dem Applikationskopf (2) und dem Griff (1) umfasst.

13. Hautbehandlungsvorrichtung nach einem der Ansprüche 2 bis 11, **dadurch gekennzeichnet, dass** das wenigstens eine Wärmeübertragungssystem (6, 6', 6"; 14) einen flexiblen Balgbereich (13) zwischen dem Applikationskopf (2) und dem Griff (1) umfasst.

14. Hautbehandlungsvorrichtung nach einem der vorstehenden Ansprüche, **dadurch gekennzeichnet, dass** die Wärmequelle eine oberflächenemittierende Laseranordnung mit vertikalem Hohlraum (VCSEL), eine oberflächenemittierende Laseranordnung mit vertikalem Außenhohlraum (VECSEL), eine lichtemittierende Diodenanordnung (LED) oder eine organische lichtemittierende Diodenanordnung (OLED) umfasst.

## Revendications

1. Dispositif de traitement de la peau comprenant
un manche (1) comprenant une alimentation en énergie,
une tête d'application (2) comprenant au moins une source de chaleur comprenant au moins une source de lumière semi-conductrice (3), et
au moins un dissipateur thermique comprenant des ailettes de refroidissement (4),
dans lequel le dissipateur thermique est situé éloigné de la source de chaleur à l'intérieur du manche (1),
dans lequel la source de chaleur est raccordée thermiquement au dissipateur thermique au moyen d'un système de transfert thermique (6, 6', 6") qui comprend au moins un corps tubulaire scellé hermétiquement contenant un milieu de travail, dans lequel la pression à l'intérieur du corps tubulaire est choisie de telle sorte que le milieu de travail est un liquide saturé à la température ambiante et peut être vaporisé par la source de chaleur,
et dans lequel la tête d'application (2) est scellée sans ouvertures à l'ambiance, **caractérisé en ce que** la tête d'application (2) comprend au moins un canal d'air (9a, 9b, 9c) adapté pour guider l'air vers la au moins une source de lumière semi-conductrice (3), le au moins un canal d'air (9a, 9b, 9c) ayant au moins une ouverture d'entrée (8) qui est située dans le manche (1) et au moins une ouverture de sortie (10) qui est située dans le manche (1).

2. Dispositif de traitement de la peau selon la revendication 1, **caractérisé en ce que** ledit au moins un canal d'air (9a, 9b, 9c) est adapté pour guider l'air à travers un trou (9c) entre la au moins une source de lumière semi-conductrice (3) et une fenêtre transparente ou translucide en contact avec la peau (7) de la tête d'application (2).

3. Dispositif de traitement de la peau selon l'une quelconque des revendications précédentes, comprenant une fenêtre en contact avec la peau (7), **caractérisé en ce que** la fenêtre en contact avec la peau (7) est raccordée thermiquement au système de transfert thermique (6) ou est raccordée thermiquement à un système de transfert thermique supplémentaire (14) qui comprend un corps tubulaire scellé hermétiquement contenant un milieu de travail dans lequel la pression à l'intérieur du corps tubulaire est choisie de telle sorte que le milieu de travail est un liquide saturé à la température ambiante qui peut être vaporisé par la source de chaleur.

4. Dispositif de traitement de la peau selon la revendication 3, **caractérisé en ce que** le dispositif de traitement de la peau comprend un refroidisseur thermoélectrique (16) et **en ce que** la fenêtre en contact avec la peau (7) est raccordée thermiquement au système de transfert thermique (6, 6', 6") ou au système de transfert thermique supplémentaire (14) au moyen d'un refroidisseur thermoélectrique (16).

5. Dispositif de traitement de la peau selon l'une quelconque des revendications 2 à 4, **caractérisé en ce que** la tête d'application (2) comprend des ailettes de refroidissement (12) qui sont raccordées thermiquement à la fenêtre en contact avec la peau (7).

6. Dispositif de traitement de la peau selon l'une quelconque des revendications précédentes, **caractérisé en ce que** la source de chaleur est raccordée électriquement à l'alimentation en énergie au moyen d'au moins un corps tubulaire d'un système de transfert thermique (6, 6', 6" ; 14)

7. Dispositif de traitement de la peau selon l'une quelconque des revendications précédentes, **caractérisé en ce que** le dissipateur thermique comprend en outre au moins un ventilateur de refroidissement (5).

8. Dispositif de traitement de la peau selon l'une quelconque des revendications précédentes, **caractérisé en ce que** la tête d'applicateur (2) comprend un ventilateur (11) adapté pour faire passer de l'air à travers le canal d'air (9a, 9b, 9c).

9. Dispositif de traitement de la peau selon l'une quelconque des revendications 2 à 8, **caractérisé en ce qu'un** premier côté de la au moins une source de lumière semi-conductrice (3) est raccordé thermiquement au système de transfert thermique (6, 6', 6") et qu'un deuxième côté de la au moins une source de lumière semi-conductrice (3) qui est opposé au premier côté est situé à l'intérieur de la tête d'application (2) selon une position et une orientation permettant à la lumière d'être émise depuis la au moins une source de lumière semi-conductrice (3) à travers la fenêtre en contact avec la peau (7).

10. Dispositif de traitement de la peau selon l'une quelconque des revendications 2 à 9, **caractérisé en ce que** la tête d'application (2) est raccordée de manière à pouvoir pivoter au manche (1) au moyen d'une charnière à couple constant (21).

11. Dispositif de traitement de la peau selon l'une quelconque des revendications 2 à 10, **caractérisé en ce que** la tête d'application (2) est raccordée de manière à pouvoir pivoter au manche (1) au moyen d'une charnière conductrice de chaleur (21) ayant un tourillon (22) et un vérin (23), dans lequel le tourillon (22) comprend une partie du corps tubulaire d'un système de transfert thermique (6, 6', 6" ; 14) et le vérin (23) comprend une partie du corps tubulaire d'un système de transfert thermique (6, 6', 6" ; 14) et/ou une feuille de graphite pyrolytique (24).

12. Dispositif de traitement de la peau selon l'une quelconque des revendications 2 à 11, **caractérisé en ce que** le au moins un système de transfert thermique (6, 6 ', 6" ; 14) comprend une feuille de graphite pyrolytique (24) entre la tête d'application (2) et le manche (1).

13. Dispositif de traitement de la peau selon l'une quelconque des revendications 2 à 11, **caractérisé en ce que** le au moins un système de transfert thermique (6, 6 ', 6" ; 14) comprend une section de soufflets souple (13) entre la tête d'application (2) et le manche (1).

14. Dispositif de traitement de la peau selon l'une quelconque des revendications précédentes, **caractérisé en ce que** la source de chaleur comprend un ensemble diode laser à cavité verticale émettant par la surface (VCSEL), un ensemble diode laser à cavité verticale externe émettant par la surface (VECSEL), un ensemble diode électroluminescente (LED) ou un ensemble diode électroluminescente organique (OLED).
